# EUROPEAN PATENT APPLICATION

(11) **EP 3 326 512 A1**
(43) Date of publication of application: **30.05.2018**
(21) Application number: 16827306.8
(22) Date of filing: 20.07.2016
(51) Int. Cl.: A61B 3/02, A61B 3/107, A61B 3/113

(54) **ELECTRO-OPTICAL DEVICE FOR EXPLORING THE BEHAVIOUR OF THE EYES IN RESPONSE TO EXTERNAL VISUAL STIMULI**

(30) Priority: 21.07.2015 ES 201531073
(71) Applicant: Davalor Salud S.L., 31192 Tajonar (Navarra) (ES); Universitat Politècnica De Catalunya, 08034 Barcelona (ES)
(72) Inventor: PUJOL RAMO, Jaume, 08232 Viladecavalls (Barcelona) (ES); ARASA MARTÌ, José, 08232 Viladecavalls (Barcelona) (ES); VILASECA RICART, Meritxell, 08222 Terrasa (Barcelona) (ES); ARJONA CARBONELL, María Montserrat, 08232 Viladecavalls (Barcelona) (ES)
(74) Representative: Cabinet Plasseraud
(86) International application number: PCT/ES2016/070549
(87) International publication number: WO 2017/013296

(57) **Abstract**

An electro-optical device for exploring the behavior of the eyes in response to external visual stimuli, which comprises a transparent mirror (2) provided with a coating that reflects visible light and allows infrared and ultraviolet light to pass through, the mirror (2) being inclined in an axial direction (3) that coincides with the gaze direction of the eye (1). Situated behind the mirror (2) are an optical system (4) provided with a sensor that captures infrared and ultraviolet light, and a light source (5) that projects an infrared or ultraviolet light emission (8) to the eye (1), the eye emitting a reflection to the optical system (4) in the axial direction (3), while a projection of images (6) in visible light is projected from one side onto the inner face of the mirror (2), the image being reflected to the eye (1) according to a projection in the same axial direction (3).

## Description

### Technical field

The present invention is related to exploring the behavior of the eyes to detect functional problems that they may have, proposing an electro-optical device that enables capturing under advantageous conditions the movement of the eyes in response to external visual stimuli of images presented before them.

### State of the art

Vision is the gateway of 95% of the external information we receive; therefore, it is the most important route for spatial location, emotional communication and, particularly, learning (for example, with regard to skills of formal recognition, reading, reading comprehension, etc.), such that it is advisable to periodically explore vision to detect and treat the functional problems that may arise.

For said vision exploration, devices for capturing the movement of the eyes in response to external visual stimuli by means of images presented before them are usually used, such as the device of Patent ES 2444542, which belongs to the same owner of the present invention, in which there are means for emitting light onto the eye and means for capturing the light that the eye reflects, coordinating a system for presenting visual stimuli before the eye with these functions in order to process the information of said means with a computer, wherein the topography of the eye is determined.

Said devices have the problem that the means for capturing the light that the eye reflects and the system for presenting visual stimuli before the eye cannot both be aligned with the gaze direction of the eye, meaning that the means for capturing the light reflected by the eye are misaligned in the gaze direction, guiding the light that is reflected by the eye to said means by means of mirrors, which diminishes the operational efficiency of the device and limits the width of the space that can be observed on the eye.

### Object of the invention

According to the invention, an electro-optical device is proposed, based on an embodiment that enables alignment with the gaze direction of an eye, for a presentation of images to create visual stimuli, as well as an optical system for capturing the light that the eye reflects from a light emitted onto it by means of a light source.

This device object of the invention comprises a mirror, the structure of which reflects visible light (400 - 800 nm) but allows non-visible light of the infrared range (800 - 1200 nm) and of the ultraviolet range (300 - 400 nm) to pass through, with said mirror arranged in an inclined position in an axial direction in which an optical system for capturing non-visible infrared and ultraviolet light is arranged behind the mirror, while a light source for emitting non-visible light is arranged behind the mirror, in an offset position in said axial direction in which the optical system for capturing non-visible light is found, and is oriented to project said light to an area located in front of the mirror in which it runs in the axial direction in which the optical system for capturing non-visible light is found.

With regard to this assembly, a projection of images in visible light is provided, projected from one side to an area of the inner face of the mirror, such that it passes the axial direction in which the optical system for capturing non-visible light is found.

Thus, a device is obtained which, in being arranged in front of an eye, the gaze direction of which coincides with the axial direction in which the optical system for capturing non-visible light is found, and which is located in the confluence of said axial direction with the projection of non-visible light coming from the light source, the images that are projected onto the inner face of the mirror are reflected to the eye in the gaze direction thereof, as the non-visible light that the eye reflects passes through the mirror in the same gaze direction, being captured in that same direction by the capturing optical system.

Thus, axial coincidence with respect to the eye is achieved for the presentation of images that create visual stimuli, as well as for the capture of the light that the eye reflects during the stimuli, thus enabling exploration of the functional behavior of the eye to be carried out with utmost efficiency and also in an area of exploration that is large enough to cover the entire field of eye movement.

Therefore, the recommended device is the result of very advantageous characteristics for the purpose of exploring the functional behavior of the eyes, for which it is intended, taking on a life of its own and having a preferential nature with regard to the conventional devices of the same application.

### Description of the figures

Figure 1 schematically shows an example of the electro-optical device object of the invention, with regard to an eye of application.
Figure 2 schematically shows the projection of the images of visual stimuli onto the eye of application in the electro-optical device.
Figure 3 shows the projection of non-visible light onto the eye of application in the electro-optical device.
Figure 4 shows the capture of the reflections of non-visible light from the eye of application in the electro-optical device.

### Detailed description of the invention

The object of the invention relates to an electro-optical device intended to explore the functional behavior of an eye (1) before visual stimuli provided by means of the presentation of images in front of the same. The device is based on the use of a structured mirror (2) such that it reflects visible light (400 - 800 nm), but it allows non-visible light from both the infrared range (800 - 1200 nm) and the ultraviolet range (300 - 400 nm) to pass through. To do so, a mirror (1) formed by a body of transparent material, such as "Crown" or "Flint" glass, borosilicate or quartz, with an inner coating is used, for example, of the type known as "cold mirror".

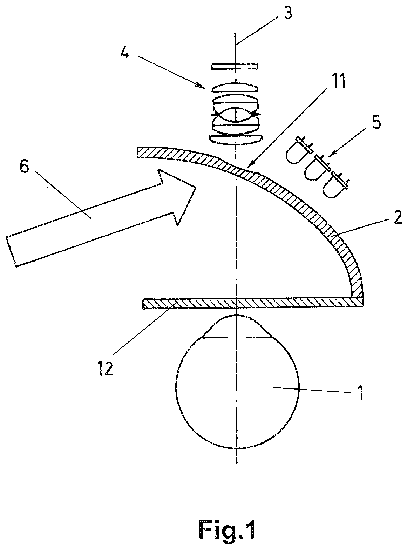

As observed in Figure 1, the mirror (2) is arranged in an inclined position in an axial direction (3) in which an optical system (4) that includes a CMOS or CCD-type sensor capable of capturing non-visible infrared and ultraviolet light is arranged behind said mirror (2).

There is also a light source (5) for emitting non-visible light arranged behind the mirror (2), in an offset position of the axial direction (3), oriented toward an area in which the focusing direction thereof runs in the axial direction (3) in front of the mirror (2); while from a lateral position a projection of images (6) guided to the area where the axial direction (3) passes through the mirror (2) is projected from an optical emitter (not shown).

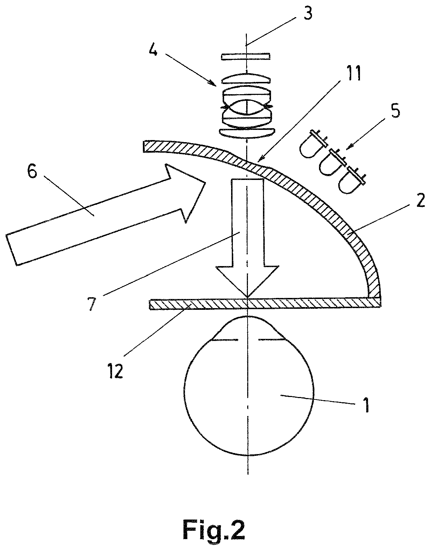

Under these conditions, by arranging the electro-optical device in front of an eye (1), such that the eye (1) remains in the area of confluence of the focusing direction of the light source (5) with the axial direction (3) and the gaze direction of the eye (1) coincides with the axial direction (3), the projection of images (6) that is projected onto the inner face of the mirror (2) is reflected according to a projection (7) to the eye (1) in the gaze direction thereof, as observed in Figure 2.

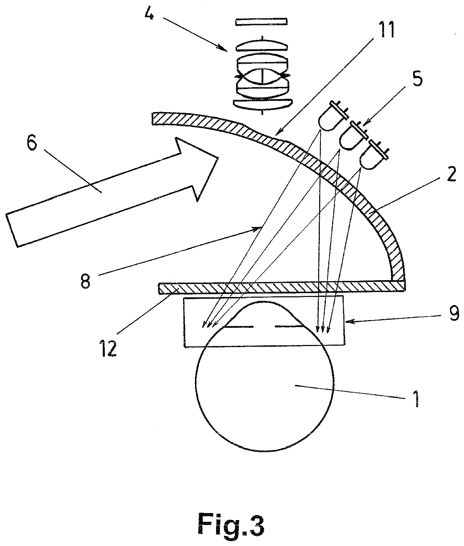

Furthermore, the non-visible light emission (8) that the light source (5) emits is projected to an area (9) where the front part of the eye (1) is located, as shown in Figure 3, such that said area (9) of projection covers the entire field of movement of the eye (1).

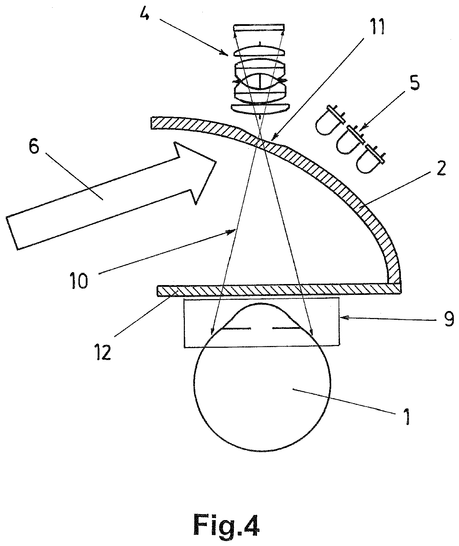

Thus, the eye (1) emits a reflection (10) of the non-visible light received, said reflection (10) being projected in the axial direction (3) to the optical system (4) through the mirror (2), as observed in Figure 4.

In doing so, the projection of the reflection (10) that the eye (1) emits to the capturing optical system (4) and the projection (7) of the images of optical stimulus to the eye (1) are both produced in the axial direction (3), and therefore, in the gaze direction of the eye (1). This enables a result of the exploration of the eye (1) to be obtained in order to determine the more specific functional behavior thereof, along with the conventional systems where the reflections of the eye of application are captured from a position that is not aligned with the gaze of the eye.

The use of non-visible light from the infrared range (800 - 1200 nm) enables algorithms that go beyond the geometric determination of the axis of the vision the eye (1) to be applied with regard to the iris of the eye (1) that is explored, such that greater exploration accuracy is obtained.

The use of non-visible light from the ultraviolet range (300 - 400 nm) enables, if applicable, an additional value to be obtained regarding the presence of bacteria in the eye (1) that is explored, since most bacteria are fluorescent in this range of non-visible light.

In the corresponding area in front of the optical system (4), a structure (11) is envisaged in the external face of the mirror (2) that makes a determination like a magnifying glass, which favors the capture of the reflection (10) emitted by the eye (1) by the optical system (4), thus making the operation of the optical device more efficient.

With regard to the non-visible light emission (8) from the light source (5) and with regard to the reflection (10) that the eye (1) emits to the optical system (4), complementary polarization elements, such as lenses or prisms (not shown), can also be arranged in the outer part of the mirror (2), which achieves a higher quality of the image of the eye (1) that is obtained in the optical system (4) in order to determine the functional behavior of vision.

In the recommended electro-optical device, the incorporation of a transparent screen (12) is envisaged between the position of the eye (1) of application and the mirror (2) of the device, which provides protection in order to prevent the entry of dust or another type of dirt into the device, constituting a surface that is easy to clean when necessary.

## Claims

1. An electro-optical device for exploring the behavior of the eyes in response to external visual stimuli, comprising a light source that emits a projection of light onto the eye (1) and an optical system that receives the light reflected in the eye (1) **characterized in that** it comprises a mirror (2) made up of a transparent body provided with a coating that reflects visible light (400 - 800 nm) and allows non-visible light of the infrared range (800 - 1200 nm) and of the ultraviolet range (300 - 400 nm) to pass through, said mirror (2) being arranged in an inclined position in an axial direction (3) that coincides with the gaze direction of the eye (1), in which an optical system (4) provided with a sensor that captures non-visible infrared and ultraviolet light is located behind the mirror (2) with respect to the position of the eye (1), a light source (5) that projects a non-visible infrared or ultraviolet light emission (8) to the eye (1), the eye emitting a reflection to the optical system (4) in the axial direction (3), while a projection of images (6) in visible light is projected from one side onto the inner face of the mirror (2), the image being reflected to the eye (1) according to a projection in the same axial direction (3).

2. The electro-optical device for exploring the behavior of the eyes in response to external visual stimuli according to claim 1, **characterized in that** in front of the optical system (4), the rear face of the mirror (2) has a structure (11) that makes a determination like a magnifying glass.

3. The electro-optical device for exploring the behavior of the eyes in response to external visual stimuli according to any one of the preceding claims, **characterized in that** with regard to the non-visible light emission (8) from the light source (5) and with regard to the reflection (10) that the eye (1) emits to the optical system (4), complementary polarization elements such as lenses or prisms are arranged in the outer part of the mirror (2).

4. The electro-optical device for exploring the behavior of the eyes in response to external visual stimuli according to any one of the preceding claims, **characterized in that** a transparent screen (12) to protect against dirt is arranged between the position of the eye (1) to be explored and the mirror (2).

5. The electro-optical device for exploring the behavior of the eyes in response to external visual stimuli according to any one of the preceding claims, **characterized in that** the non-visible light emission (8) from the light source (5) is projected onto the eye (1) of application in an area (9) that covers the entire field of movement thereof.
